# EUROPEAN PATENT APPLICATION

(11) **EP 1 669 338 A1**
(43) Date of publication of application: **14.06.2006**
(21) Application number: 03751309.0
(22) Date of filing: 01.10.2003
(51) Int. Cl.: C07C 37/82, C07G 5/00, A61K 31/352, A61K 36/18, C07D 311/62

(54) **PROCESS FOR PRODUCING PRODUCT CONTAINING PROANTHOCYANIDIN IN HIGH PROPORTION**

(71) Applicant: Toyo Shinyaku Co., Ltd., Fukuoka-shi, Fukuoka 812-0011 (JP)
(72) Inventor: TAKAGAKI, Kinya c/o Toyo Shinyaku Co., Ltd., shi, Fukuoka 812-0011 (JP); YAMAGUCHI, Gotaro c/o Toyo Shinyaku Co., Ltd., shi, Fukuoka 812-0011 (JP)
(74) Representative: Arth, Hans-Lothar
(86) International application number: PCT/JP2003/012620
(87) International publication number: WO 2005/033053

(57) **Abstract**

A proanthocyanidin-containing product containing a large amount of highly bioactive OPCs can be obtained easily by treating an extract or juice of a plant with at least two types of adsorbents that differ from one another in at least one of material, pore radius, specific surface area, and an ability of adsorbing and releasing a substance based on the molecular weight of the substance.

## Description

### Technical Field

The present invention relates to a method for producing efficiently a proanthocyanidin-containing product containing a large amount of highly bioactive OPCs.

### Background Art

Proanthocyanidins are tannins that are present in various plants. Proanthocyanidins are a group of compounds that are condensation or polymerization products (hereinafter, referred to as "condensation products") and have flavan-3-ol and/or flavan-3,4-diol as a constituent unit. When these compounds are subjected to acid treatment, anthocyanidins such as cyanidin, delphinidin, and pelargonidin are produced. Therefore, these compounds are designated as proanthocyanidins.

Proanthocyanidins, which are one type of polyphenol, are potent antioxidants produced by plants, and are contained concentratedly in portions of plant leaves, bark, or skin or seeds of fruits. More specifically, proanthocyanidins are contained in, for example, the seeds of grape; the bark of pine; the skin of peanuts; the leaves of ginkgo; the fruit of locust; and the fruit of cowberry. Moreover, it is known that proanthocyanidins are also contained in cola nuts in West Africa; the roots of Rathania in Peru; and Japanese green tea. Proanthocyanidins cannot be produced in the human body.

Proanthocyanidins generally can be obtained by extraction from plants. Examples of the solvent used for the extraction include water; organic solvents such as methanol, ethanol, acetone, hexane, and ethyl acetate; or mixtures of these solvents (Japanese Laid-Open Patent Publication No. 11-80148). However, when simply performing an extraction with a solvent, the amount of proanthocyanidins that can be recovered is small and also the purity of the resultant extract is low. Therefore, in order to use the extract as raw materials for health food products, cosmetics, and pharmaceuticals, it is necessary to increase the purity. Thus, additional processes such as concentration and purification are required, which increases the cost and time.

Methods for recovering polyphenols containing proanthocyanidins have been reported. For example, Japanese Laid-Open Patent Publication Nos. 5-279264 and 6-56689 describe a process of adsorbing polyphenols to a chitin substrate and that the chitin substrate to which the polyphenols are adsorbed is utilized as a polyphenol product. Japanese Laid-Open Patent Publication No. 2002-97187 describes a method for recovering free polyphenols that comprises adding ascorbic acid and an alkali metal or a salt thereof to a plant extract liquid in order to adjust the pH in the range of 6 to 11, thereby precipitating a metal salt of polyphenols, recovering the metal salt, desalting the metal salt with an ion exchange resin or the like, and recovering the resultant free polyphenols.

In recent years, it has been reported that among the proanthocyanidins, in particular, condensation products having a degree of polymerization of 2 to 4 (dimer to tetramer) have an excellent antioxidation ability. In this specification, the condensation products having a degree of polymerization of 2 to 4 are referred to as "oligomeric proanthocyanidins" (OPCs). In addition to the antioxidation ability, OPCs are also known to provide, for example, an effect of inhibiting bacterial proliferation in the oral cavity to reduce plaque (dental plaque); an effect of recovering the elasticity of blood vessels; an effect of preventing lipoprotein in blood from being damaged by active oxygen, thereby preventing aggregation and adherence of the oxidized fats onto the inside wall of the vessel, thus preventing cholesterol from being aggregated and adhered onto the oxidized fats that have been adhered onto the inside wall of the vessel; an effect of regenerating vitamin E that has been degraded by active oxygen; and an effect of serving as an enhancer of vitamin E.

However, most of the proanthocyanidins that are obtained by the above-described methods have a high degree of polymerization, and the content of highly bioactive OPCs (i.e., condensation products having a degree of polymerization of 2 to 4) is very low.

Japanese Laid-Open Patent Publication Nos. 4-190774, 10-218769, and 2001-131027 and Eberhard Scholz et al., "Proanthocyanidins from Krameria triandra Root," Planta Medica, 55(1989), pp. 379-384 describe methods for extracting OPCs from plants and/or synthesizing OPCs. In the extraction methods, an extract liquid of a plant is brought into contact with an adsorbent, the adsorbed material is eluted, and specific fractions are collected, and thereafter the same process is repeated using the collected fractions. Without repeating the process, an OPC-containing product having a high OPC content cannot be obtained, which is not efficient. The synthesizing methods also include a lot of steps, which causes problems of high cost and long time. Furthermore, the synthesizing methods also have a problem of liquid waste disposal.

Therefore, there is a demand for a method for purifying proanthocyanidins containing a large amount of OPCs.

### Disclosure of Invention

The inventors of the present invention conducted in-depth studies on a method for efficiently obtaining a proanthocyanidin-containing product that contains useful highly bioactive OPCs at a high ratio. As a result, the inventors found that a proanthocyanidin-containing product that contains a large amount of highly bioactive OPCs can be obtained efficiently by treating an extract or juice of a plant with at least two types of adsorbent, and thus the present invention was achieved.

A method of the present invention for producing a proanthocyanidin-containing product comprises the step of treating an extract or juice of a plant with at least two types of adsorbents, wherein the adsorbents differ from one another in at least one of material, pore radius, specific surface area, and an ability of adsorbing and releasing a substance based on the molecular weight of the substance.

In a preferred embodiment, at least one of the adsorbents is a synthetic adsorbent.

In a preferred embodiment, the method is performed using two types of adsorbents, wherein a first adsorbent is a synthetic adsorbent, and a second adsorbent is selected from the group consisting of a synthetic adsorbent, a cation exchange resin, an anion exchange resin, a crosslinked dextran derivative, a polyvinyl resin, an agarose derivative, and a cellulose derivative.

In a preferred embodiment, at least one of the adsorbents can remove a proanthocyanidin having a degree of polymerization of 5 or more or impurities from the extract or juice of a plant.

In a preferred embodiment, at least one of the adsorbents is porous and has a pore radius of not more than 90 Å or not less than 100 Å.

In a preferred embodiment, at least one of the adsorbents has an ability of adsorbing or releasing a substance having a molecular weight in the range of 100 to 20000.

### Best Mode for Carrying Out the Invention

The method of the present invention for producing a proanthocyanidin-containing product comprises the step of treating an extract or juice of a plant with at least two types of adsorbents, and the at least two types of adsorbents differ from one another in at least one of material, specific surface area, pore radius, and the ability of adsorbing and releasing a substance based on the molecular weight of the substance. The proanthocyanidin-containing product obtained by the production method of the present invention contains OPCs abundantly, wherein the OPCs are proanthocyanidins having a degree of polymerization of 2 to 4.

### (Extract or juice of plant)

First, an extract or juice of a plant is obtained.

There is no limitation on the type of the plant used in the present invention, as long as the plant contains proanthocyanidins. Examples of the plant include the bark of plants such as Cryptomeria japonica, white cedar, and pine; the fruit, fruit skin, and seeds of plants such as grape, blueberry, strawberry, avocado, locust, cowberry, and elderberry; barley; wheat; soybean; black soybean; cacao; adzuki bean; the hull of conker; the inner skin of peanuts; the leaves of ginkgo; tea leaves and tea extract liquid; sorghum; apple fruits; Sasa veitchii; fucoidan; yacon leaves; cola nuts (e.g., cola nuts in West Africa); and the roots of Rathania (e.g., Rathania in Peru). Among these, in particular, pine bark, the seeds and fruit skin of grape, and the inner skin of peanuts are preferably used.

In order to obtain an extract of a plant, an extraction solvent is added to the plant, and the mixture is kept at a predetermined temperature as necessary.

When performing extraction, in view of the extraction efficiency, it is preferable that a plant is pulverized to an appropriate size to increase the surface area per volume. There is no limitation on the method for pulverization. For example, a pulverized product obtained by the use of a cutter, slicer, or the like; or a ground product obtained by the use of a mixer, juicer, blender, masscolloider, or the like can be employed. The pulverized product or the ground product is a small piece having a size of 0.01 to 10 cm, preferably 0.01 to 5 cm. In order to increase the pulverization efficiency, water or an organic solvent such as ethanol, methanol, or ethyl acetate may be added at the time of pulverization.

As the extraction solvent, water or an organic solvent is used. Examples of the organic solvent include methanol, ethanol, 1-propanol, 2-propanol, 1-butanol, 2-butanol, acetone, hexane, cyclohexane, propylene glycol, aqueous ethanol, aqueous propylene glycol, methyl ethyl ketone, glycerin, methyl acetate, ethyl acetate, diethyl ether, dichloromethane, edible oils or fats, 1,1,1,2-tetrafluoroethane, and 1,1,2-trichloroethene. Furthermore, a mixed solvent of water and an organic solvent may be preferably used. These organic solvents can be used alone or in combination. In view of the disposal of liquid waste formed in the production process, or in view of a treatment of adding a metal or metal salt described later, water is preferably used.

The amount of the extraction solvent to be added to the plant can be determined in view of the desired concentration of proanthocyanidins and the extraction efficiency. For example, when water is used as the extraction solvent, the weight ratio of the plant and water is 1:5 to 1:100, preferably 1:10 to 1:50. When water and/or an organic solvent is added to perform pulverization, the amount of the extraction solvent to be added can be determined by taking the amount of water and/or the organic solvent used for the pulverization into account.

A higher extraction temperature is preferable in order to increase the extraction efficiency. For example, when water is used, the extraction is performed with hot water at 50°C to 120°C, preferably 70°C to 100°C. Hot water may be added to the plant, and after water is added to the plant, the mixture can be heated. The extraction time is determined as appropriate, depending on the extraction temperature. The extraction time is generally 10 minutes to 48 hours, preferably 30 minutes to 24 hours. When the mixture is heated as described above, the mixture is recommended to be further allowed to stand at a temperature of 0°C to 30°C for 1 to 2 days. This is because proanthocyanidins, which are difficult to elute from plants, can be extracted efficiently.

As the method for extraction with an organic solvent, a heat extraction method or supercritical fluid extraction method can be performed. As heat extraction, a process of adding a warmed solvent to the plant, or a process of adding a solvent to the plant and then heating the resultant mixture can be employed. For example, a water-ethanol mixed solvent comprising water and ethanol at a weight ratio of 1:1 to 1:9 is used as the extraction solvent in an amount of 1 to 20 times the amount of the pulverized plant. Then, the extraction is performed by stirring the resultant mixture for 0.5 hours to 6 hours while being refluxed at 70°C to 75°C. When the temperature of the extraction is not raised to the temperature of reflux, the extraction efficiency can be increased by a process including the steps of performing a heat extraction once with the mixed solvent, recovering the supernatant from the resultant mixture by filtration or the like, adding the mixed solvent to the remaining residue, and warming the resultant mixture. When an organic solvent is used, it is necessary to set the extraction temperature to not more than the boiling point of that organic solvent.

Supercritical fluid extraction is a method for extracting a target component using a supercritical fluid. A supercritical fluid is in a state that is above the liquid-vapor critical point in the phase diagram showing critical temperature and critical pressure. Examples of compounds that can be employed as a supercritical fluid include carbon dioxide, ethylene, propane, and nitrous oxide (laughter gas). Carbon dioxide is preferably used.

Supercritical fluid extraction includes an extraction step in which a target component is extracted with a supercritical fluid and a separation step in which the target component is separated from the supercritical fluid. In the separation step, any separation process can be employed, examples of which include a separation based on a change in pressure, a separation based on a change in temperature, and a separation using an adsorbent or absorbent.

Moreover, it is also possible to perform supercritical fluid extraction in which an entrainer is added. In this method, supercritical fluid extraction is performed using an extracting fluid obtained by adding, for example, ethanol, propanol, n-hexane, acetone, toluene, or another aliphatic lower alcohol, aliphatic hydrocarbon, aromatic hydrocarbon, or ketone at about 2 to 20 w/v% to a supercritical fluid, so that the solubility of a target substance to be extracted, such as OPCs and catechins (described later), in the extracting fluid is dramatically increased or the selectivity of separation is enhanced. Thus, proanthocyanidins are obtained efficiently.

For extraction, any extraction apparatus, for example, a batch type, semi-continuous, or continuous extraction apparatus can be used.

In order to obtain juice of a plant, the plant is directly squeezed, or cut or pulverized as appropriate and then squeezed. This method is preferably employed when a plant having a high water content is used. For example, in the case of the fruit of grape, juice containing proanthocyanidins can be obtained by squeezing. A plant pulverized product (e.g., pulverized product of the fruit of grape described above) obtained by pulverizing a plant and containing a solid derived from the plant can be used as well as the juice. In this specification, the plant pulverized product containing a solid derived from a plant is included in the juice.

### (Adsorbent)

In the method of the present invention, at least two types of adsorbents are employed as described above. There is no limitation on each adsorbent used in the present invention, as long as the adsorbent is a filler that is commonly used for column chromatography. Examples of the adsorbent include synthetic adsorbents, cation exchange resins, anion exchange resins, crosslinked dextran derivatives, polyvinyl resins (used in gel form), agarose derivatives, cellulose derivatives, silica gel, and silica gel used for reverse phased chromatography. Synthetic adsorbents, cation exchange resins, anion exchange resins, crosslinked dextran derivatives, polyvinyl resins (used in gel form), agarose derivatives, cellulose derivatives, and the like are preferable. Synthetic adsorbents are more preferable. Here, synthetic adsorbents refer to adsorbents made of a compound that does not have a functional group such as an ion exchange group and that are porous and have fine continuous holes (pores). Such adsorbents are capable of adsorbing a substance by the van der Waals force, for example.

The synthetic adsorbents are classified into aromatic synthetic adsorbents, substituted aromatic synthetic adsorbents, and acrylic synthetic adsorbents according to their materials. The degree of hydrophilicity and hydrophobicity varies among these synthetic adsorbents depending on the material thereof. In view of the stability, efficiency of adsorbing proanthocyanidins (OPCs), and separation or fractionation ability of the synthetic adsorbents, aromatic synthetic adsorbents are preferable.

The aromatic synthetic adsorbents are porous and made of a crosslinked styrene resin and the like. Examples of commercially available such adsorbents include DIAION (registered trademark) HP-10, HP-20, HP-21, HP-30, HP-40, and HP-50 (manufactured by Mitsubishi Chemical Corporation); Amberlite (registered trademark) XAD-4, XAD-16, XAD-1180, and XAD-2000 (manufactured by ORGANO CORPORATION); and SEPABEADS (registered trademark) SP-825, SP-800, SP-850, and SP-875 (manufactured by Mitsubishi Chemical Corporation).

The substituted aromatic synthetic adsorbents are adsorbents made of a resin with strong hydrophobicity that is an aromatic polymer having a bromine atom or the like at its aromatic rings. Examples of commercially available such adsorbents include SEPABEADS (registered trademark) SP-205, SP-206, and SP-207 (manufactured by Mitsubishi Chemical Corporation).

The acrylic synthetic adsorbents are adsorbents made of a resin with strong hydrophilicity that has a methacrylate polymer or the like as the framework. Examples of commercially available such adsorbents include DIAION (registered trademark) HP1MG and HP2MG (manufactured by Mitsubishi Chemical Corporation); and Amberlite (registered trademark) XAD-7 (manufactured by ORGANO CORPORATION).

Since each of the foregoing synthetic adsorbents is porous and has fine continuous holes (pores) as described above, a target solute present in a solution can be separated by each of the adsorbents according to the molecular sieve effect. Namely, when a synthetic adsorbent is brought into contact with a solution, solute molecules having a small size pass through the pores of the synthetic adsorbent, permeate and diffuse into the inside of the synthetic adsorbent, and are adsorbed thereto. On the other hand, molecules having a larger size than the pores cannot diffuse into the synthetic adsorbent and thus are not adsorbed thereto.

Examples of the adsorbent made of a cation exchange resin include Amberlite (registered trademark) CG-4000, CG-5000, CG-6000, CG-8000, IR-116, IR-118, IR-120B, IR-122, IR-124, XT-1007, XT-1009, and XT-1002 (manufactured by ORGANO CORPORATION), which are resins having a sulfonate group as a functional group.

Examples of the adsorbent made of an anion exchange resin include OPTIPORE-XUS 40285.00 and OPTIPORE-XUS 40390.00 (manufactured by The Dow Chemical Company), which are weak basic anion exchange resins having a quaternary ammonium group as a functional group.

Examples of the adsorbent made of a crosslinked dextran derivative include Sephadex (registered trademark) LH20 and LH60 (manufactured by Amersham Biosciences K.K.).

Examples of the adsorbent made of a polyvinyl resin (gel) include TOYOPEARL HW-40 and 50 (manufactured by TOSOH Corporation).

Examples of the adsorbent made of an agarose derivative include Sepharose CL, 4B and 6B (Amersham Biosciences K.K.) and Bio-Gel A (Bio-Rad Laboratories, Inc.).

Examples of the adsorbent made of a cellulose derivative include CELLULOFINE CL-90, GCL-300, and GCL-1000 (SEIKAGAKU CORPORATION).

Among the foregoing adsorbents, an adsorbent that is porous and made of a polymer having a network molecular structure is particularly preferable. For example, an adsorbent that is a synthetic adsorbent, such as DIAION HP-20 and Amberlite XAD-4, and an adsorbent made of a crosslinked dextran derivative, such as Sephadex LH20 and LH60, are particularly preferable.

The at least two types of adsorbents described above differ from one another in at least one of the material, the pore radius, the specific surface area, and the ability of adsorbing and releasing a substance based on the molecular weight of the substance. For example, adsorbents having different pore radiuses, specific surface areas, or the abilities of adsorbing and releasing a substance based on the molecular weight of the substance are regarded as different types of adsorbents even when the adsorbents are made of the same material. It is preferable that at least one of the adsorbents used is an adsorbent that can remove proanthocyanidins having a degree of polymerization of 5 or more or an adsorbent that can remove impurities, and it is more preferable that both of these adsorbents are employed in combination.

The adsorbent that can remove proanthocyanidins having a degree of polymerization of 5 or more (hereinafter this adsorbrnt referred to as "first adsorbent") as mentioned above is specifically an adsorbent that is capable of adsorbing OPCs effectively and that hardly adsorbs proanthocyanidins having a degree of polymerization of 5 or more. The adsorbent that can remove impurities (hereinafter this adsorbent is referred to as "second adsorbent") is specifically an adsorbent that adsorbs proanthocyanidins easily and that hardly adsorbs impurities. Preferably, the adsorbent is one that has such properties and furthermore that, after adsorbing proanthocyanidins, allows OPCs to be selectively released or separated from the adsorbed proanthocyanidins and to elute the OPCs, thereby providing a solution having a high OPC content.

When the first adsorbent and the second adsorbent are selected based on the material, an aromatic synthetic adsorbent is preferably selected as the first adsorbent. The second adsorbent is preferably selected from the group consisting of a synthetic adsorbent, a cation exchange resin, an anion exchange resin, a crosslinked dextran derivative, a polyvinyl resin, an agarose derivative, and a cellulose derivative. More preferably, a synthetic adsorbent or a crosslinked dextran derivative, particularly preferably an aromatic synthetic adsorbent or a crosslinked dextran derivative is selected.

As the adsorbents, porous synthetic adsorbents having pores are preferable, and the first adsorbent and the second adsorbent can be selected based on the radius of the pores. This is because the size of molecules to be adsorbed or the adsorption ability of the adsorbent to the molecules varies depending on the pore radius.

The first adsorbent preferably has a pore radius of not more than 90 Å, more preferably 20 Å to 90 Å, and even more preferably 30 Å to 80 Å. An adsorbent having a smaller pore radius has a higher adsorption ability to low molecular weight molecules (having a molecular weight of several thousands or less) rather than to high molecular weight molecules. Thus, while a compound with a low molecular weight is adsorbed, proanthocyanidins having a degree of polymerization of 5 or more, which have a relatively high molecular weight, can be removed without being adsorbed. As such an adsorbent, for example, SEPABEADS SP-825, SEPABEADS SP-850, Amberlite XAD-4, and XAD-2000, which are aromatic synthetic adsorbents, are preferable.

The second adsorbent preferably has a pore radius of not less than 100 Å, more preferably 100 Å to 500 Å, and even more preferably 100 Å to 300 Å. An adsorbent having a larger pore radius efficiently adsorbs proanthocyanidins with a wider molecular weight range of from several thousands to several tens of thousands, but has a weaker adsorptive ability for impurities, and therefore does not adsorb the impurities. Thus, the impurities can be removed easily. As such an adsorbent, for example, DIAION HP-20, DIAION HP-21, and Amberlite XAD-16, which are aromatic synthetic adsorbents, or Sephadex LH20, which is an adsorbent made of a crosslinked dextran derivative, is preferable.

When the first adsorbent and the second adsorbent are selected based on the specific surface area, it is preferable that both of the first adsorbent and the second adsorbent have a specific surface area of not less than 500 m²/g in view of the adsorption ability Furthermore, in view of efficient adsorption of OPCs, it is more preferable that the first adsorbent has a specific surface area of not less than 700 m²/g. Since the specific surface area varies among adsorbent depending on the size of each of the adsorbents or depending on the size and the number of pores of each of the adsorbents in case of porous adsorbents, an adsorbent having a required specific surface area can be selected as appropriate.

When fractionation is conducted based on the molecular weight of a substance to be fractionated using an adsorbent made of a crosslinked dextran derivative, a polyvinyl resin (used in gel form), or the like as the adsorbent, there is no particular limitation on the ability of adsorbing and releasing a substance based on the molecular weight of the substance. However, it is preferable that the first adsorbent has an ability of adsorbing and releasing a substance having the molecular weight in the range of 100 to 20,000, preferably 100 to 5,000. It is preferable that the second adsorbent has an ability of adsorbing and releasing a substance having the molecular weight in the range of 100 to 20,000, preferably 100 to 10,000. Adsorbents having an ability of adsorbing and releasing a substance having such a range of the molecular weight can adsorb proanthocyanidins and remove impurities. Furthermore, OPCs in the adsorbed proanthocyanidins can be eluted and collected in fractions. As such adsorbents, Sephadex LH-20 and Sephadex LH-60 are preferable.

Among the above-described various adsorbents, Amberlite (registered trademark) XAD-4 and SEPABEADS SP825 and SP850 that are porous aromatic synthetic adsorbents having a pore radius of not more than 90 Å and a specific surface area of not less than 700 m²/g, are particularly preferable as the first adsorbent. As the second adsorbent, DIAION (registered trademark) HP-20 that is porous aromatic synthetic adsorbent having a pore radius of not less than 100 Å and a specific surface area of not less than 500 m²/g, and Sephadex LH20 that is an adsorbent made of a crosslinked dextran derivative, are particularly preferable.

The amount of the adsorbent can be determined as appropriate based on the amount of solids contained in an object to be treated (i.e., an extract or juice of a plant or a substance recovered from an adsorbent), the type of the solvent, which is described later, the type of the adsorbent, or the like. For example, the adsorbent is preferably used in an amount of 0.1 to 100 parts by weight, preferably 0.1 to 50 parts by weight with respect to 1 part by weight (dry weight) of solids contained in the object to be treated. When the amount of the adsorbent is less than 0.01 parts by weight with respected to 1 part by weight (dry weight) of the solid, the recovery of the proanthocyanidins may be low. When the amount of more than 100 parts by weight, there is a problem in that the recovery of the proanthocyanidins from the adsorbent is poor although proanthocyanidins are adsorbed sufficiently. When an adsorbent that has a high efficiency of adsorbing OPCs is used, the amount of the adsorbent may be determined based on the weight of the plant before the treatment without measuring the dry weight in the object to be treated, for the sake of simplicity of the operation. For example, when an aromatic synthetic adsorbent such as DIAION HP-20, SEPABEADS SP-825, or Amberlite XAD-16, or an adsorbent made of a crosslinked dextran derivative, such as Sephadex LH20, is employed, the adsorbent is used in an amount of 0.1 to 10 parts by weight (dry weight), preferably 0.2 to 5 parts by weight (dry weight) with respect to 1 part by weight (dry weight) of the plant before the treatment. According to the use of the adsorbent in such an amount, the object to be treated can be brought into sufficient contact with the adsorbent, and thus effective adsorption can be carried out. Specifically, when pine bark is treated with DIAION HP-20, DIAION HP-20 is used in an amount of 0.1 kg to 10 kg (corresponding to 0.15 L to 15 L by volume of the adsorbent) with respect to 1 kg of pine bark. When an adsorbent made of a crosslinked dextran derivative is employed, the adsorbent swells according to the addition of water and the volume thereof increases. Therefore, it is preferable to use the adsorbent such that the volume of the swollen adsorbent is 100 parts by volume to 1000 parts by volume, preferably 120 parts by volume to 500 parts by volume with respect to 1 part by weight (dry weight) of solids contained in a liquid to be treated.

### (Treatment with adsorbent)

According to the method of the present invention, a proanthocyanidin-containing product can be obtained by treating an extract or juice of a plant using the at least two types of adsorbents described above in combination, and furthermore conducting a treatment, such as concentration or a salt treatment, if necessary. For example, the extract or juice of a plant can be first brought into contact with the first adsorbent as described above, thereby removing proanthocyanidins having a degree of polymerization of 5 or more that are not adsorbed, and the adsorbate can be recovered with a predetermined solvent. Then, a proanthocyanidin-containing product containing OPCs at a high ratio can be obtained from the recovered substance using another adsorbent. In this case, the treatment with the first adsorbent and the treatment with another adsorbent may be performed in reverse order. Alternatively, the extract or juice of a plant can be first brought into contact with the second adsorbent, thereby removing impurities as non-adsorbates, and then a proanthocyanidin-containing product containing OPCs at a high ratio can be obtained from the recovered substance using another adsorbent. In this case, the treatment with the second adsorbent and the treatment with another adsorbent may be performed in reverse order. It is more preferable to perform the above-described treatment with the first adsorbent and treatment with the second adsorbent in combination. In this case, the treatment with the first adsorbent and the treatment with the second adsorbent may be performed in reverse order. Furthermore, it is also possible to perform a treatment with another adsorbent as appropriate, so that the extract or juice of a plant can be treated with three or more types of adsorbents.

The object to be treated (an extract or juice of a plant or a substance recovered from an adsorbent) can be brought into contact with the adsorbent by any method. For example, convenient methods such as a column chromatography method and a batch method can be employed. The column chromatography method comprises the steps of filling the adsorbent in a column and applying the object to be treated on the column. The batch method comprises the steps of adding the adsorbent to the object to be treated and removing the adsorbent after a predetermined period of time.

In order to carry out the column chromatography method, the solvent contained in the object to be treated is first substituted with a solvent suitable for the contact with the adsorbent, if necessary. This substitution can be conducted by a method including a process that is commonly used by those skilled in the art, such as drying by heating, freeze drying, vacuum concentration to dryness, or dialysis. For example, in the case of an aromatic synthetic adsorbent, such as DIAION HP-20, the solvent is substituted with water, and in the case of an adsorbent made of a crosslinked dextran derivative, such as Sephadex LH20, the solvent is substituted with ethanol. Next, the second adsorbent is filled in a column, the extract or juice of a plant or the recovered substance is applied on the column, and then water with a volume of, for example, 3 times to 10 times the volume of the adsorbent is applied on the column. Thus, saccharides and organic acids, which are impurities, are removed. Thereafter, proanthocyanidins are eluted with an appropriate solvent, which is described later. It should be noted that various conditions in the column chromatography method can be determined as appropriate depending on the adsorbent to be used. For example, when an adsorbent made of an ion exchange resin is used, it is preferable that the column temperature is set at 10°C to 120°C and the inside of the column is at atmospheric pressure or pressurized.

In order to carry out the batch method, an adsorbent with the same weight ratio as in the above-described column chromatography method is added to the object to be treated, and is brought into contact with it for 1 to 3 hours under stirring. Then, the adsorbent is recovered by filtration or centrifugation. For example, when the second adsorbent is used as the adsorbent, impurities can be removed through this operation. Next, the second adsorbent to which proanthocyanidins are adsorbed is added to an appropriate solvent (described later), and the resultant mixture is further stirred for 1 hour to 3 hours to release proanthocyanidins. Then the supernatant is obtained by filtration or centrifugation. Thus, a proanthocyanidin-containing product containing a larger amount of proanthocyanidins or OPCs can be obtained.

The elution solvent can be selected as appropriate according to the type of the adsorbent and the type of a substance to be adsorbed or eluted. For example, when using the first adsorbent, water, methanol, ethanol, ethyl acetate, chloroform, and mixed solvents of these can be used as the elution solvent. A mixed solvent of water and ethanol is preferably used in view of safety. Regarding the mixing ratio of water and ethanol, for example, in the case of an aromatic synthetic adsorbent, such as Amberlite XAD-4, Amberlite XAD-2000, SEPABEADS SP825, or SEPABEADS SP850, for removing proanthocyanidins having a degree of polymerization of 5 or more, an aqueous solution of ethanol having a relatively high concentration of 10 vol% or more, preferably 30 vol% or more, more preferably 50 vol% or more is preferably used in view of increasing the yield of OPCs when the adsorbed OPCs are eluted. This is because proanthocyanidins having a degree of polymerization of 5 or more have been removed.

When using the second adsorbent, proanthocyanidins are adsorbed thereto regardless of the molecular weight of the proanthocyanidins, so that a solvent that can selectively elute and separate or fractionate OPCs from the adsorbed proanthocyanidins is preferably selected as the eluent. A mixed solvent of water and ethanol (an aqueous solution of ethanol) is preferable. For example, in the case of an aromatic synthetic adsorbent, such as DIAION HP-20, HP-21, or XAD-16, an aqueous solution of ethanol with 10 vol% to 50 vol%, preferably 10 vol% to 30 vol% is preferably used in order to separate or fractionate a proanthocyanidin-containing product containing a large amount of OPCs from the adsorbate. Moreover, in the case of an adsorbent made of a crosslinked dextran derivative, such as Sephadex LH-20 or Sephadex LH-60, an aqueous solution of ethanol with 70 vol% or more, preferably 80 vol% or more is preferable.

When using an adsorbent made of an ion exchange resin, water is preferably used as the elution solvent.

By using different types of adsorbents as described above, the yield of proanthocyanidins having a degree of polymerization of 2 to 4 (OPCs) from the extract or juice of a plant can be increased, and the OPC content in the resultant proanthocyanidin-containing product can be increased efficiently.

### (Concentration and salt treatment)

As described above, concentration or a salt treatment may be performed, if necessary, before or after the treatments with at least two types of adsorbents, or between the treatment with an adsorbent and the subsequent treatment with another adsorbent.

By performing the concentration, the efficiency of adsorption to the adsorbent increases. Moreover, proanthocyanidins having a degree of polymerization of 5 or more may form a precipitate as a result of the concentration. When the precipitate is removed by filtration or the like, a proanthocyanidin-containing product having a more increased OPC content can be obtained from the resultant concentrate. Furthermore, when the adsorption treatment is performed using an aromatic synthetic adsorbent, it is necessary to use water as the solvent in light of the properties of the resin. Therefore, when an organic solvent is contained in the extract or juice of a plant, removal of the solvent can be performed in parallel with the concentration by performing the concentration before the treatment with the adsorbent.

The concentration can be performed by a concentration method that is commonly used by those skilled in the art, such as vacuum concentration or freeze drying. When the concentration is conducted by heating, the heating is performed at a temperature of 40°C to 100°C in order to prevent decomposition of proanthocyanidins due to heating. Vacuum concentration or freeze drying, which hardly causes decomposition of proanthocyanidins, is preferably used.

Furthermore, in order to conduct the concentration at a relatively low temperature in a short period of time, the solvent used for the extraction of the object to be treated is preferably an organic solvent such as ethanol having a lower boiling point than that of water or a mixed solvent of such an organic solvent and water. In view of safety in use as food products and pharmaceuticals, ethanol or a mixed solvent of ethanol and water is preferable.

The concentration is preferably performed after removing insoluble substances in the object to be treated, preferably by conducting filtration once, so that the extent of concentration can be controlled easily.

There is no limitation on the concentration degree of the resultant concentrate. The concentration is performed such that the volume of the concentrate is preferably 1/2 to 1/100, more preferably 1/5 to 1/70, even more preferably 1/10 to 1/50 of the volume of the object to be treated before the concentration.

In the salt treatment, by adding a salt to the object to be treated, proanthocyanidins having a high degree of polymerization are allowed to deposit as an insoluble substance such as a precipitate, and this insoluble substance can be removed. When performing the salt treatment, it is preferable to substitute the solvent in the object to be treated with water. When the solvent is substituted with water, ionization of the salt in a solution is promoted, and the proanthocyanidins having a high degree of polymerization can be removed efficiently.

There is no particular limitation on the temperature of the object to be treated at the time when the salt is added. It is preferable that the salt is added after the object to be treated is cooled to a temperature of 1°C to 40°C.

The salt to be added to the object to be treated may be any salt as long as it can ionize in a solution. Examples of the salt include monovalent metal salts, divalent metal salts, and nonmetallic salts.

Examples of the monovalent metal salts preferably include salts of alkali metals such as lithium, sodium, potassium, rubidium, cesium, and francium.

Examples of the divalent metal salts include salts of metals, the metals being beryllium, magnesium, and an alkaline-earth metal (calcium, strontium, barium, or radium).

Examples of the nonmetallic salts preferably include ammonium sulfate.

The divalent metal salts especially have a high adsorption ability to proanthocyanidins, and therefore bind to not only the proanthocyanidins having a high degree of polymerization but also OPCs to make them precipitate easily. Moreover, when a divalent metal salt is added, the pH in the solution tends to increase, which may cause an oxidation of proanthocyanidins. The divalent metal salt is added so that the pH in the solution is preferably less than 7.5, more preferably less than 6, and even more preferably not more than 5.5. When the pH is not less than 7.5, an additive (e.g., pH regulator such as ascorbic acid) for stabilizing proanthocyanidins can be added in advance. Furthermore, when a salt containing a metal (e.g., copper) that can be employed as an oxidizing agent is used as the divalent metal salt, care should be taken because proanthocyanidins may be oxidized as described above. Thus, in order to reduce bonding between OPCs and the salt as much as possible to increase the purification efficiency of OPCs, a monovalent alkali metal salt or ammonium sulfate is preferably employed.

Examples of such a monovalent metal salt include halide (chloride, bromide, etc.), phosphate, carbonate, and an organic acid salt (carboxylate such as acetate, sulfonate, etc.). Specific examples thereof include sodium chloride, sodium sulfate, sodium citrate, potassium chloride, sodium phosphate, potassium phosphate, and sodium acetate. In particular, sodium sulfate, potassium phosphate, sodium citrate, sodium chloride, and ammonium sulfate, which can be preferably used for salting-out, are preferable.

The above-described salt can be added in an amount of 3 wt% to 50 wt%, preferably 5 wt% to 45 wt% of the entire weight of the object to be treated, although the amount varies depending on the type of the salt. Moreover, preferably, when the maximum amount of the salt that can be dissolved in water is taken as 100 parts by weight, the salt is added to the concentrate in an amount corresponding to 10 to 75 parts by weight, more preferably 20 to 60 parts by weight. Moreover, when a divalent metal salt such as calcium salt or magnesium salt is employed as the salt, the salt can be added to the concentrate so that it is contained in the concentrate in an amount of 0.1 wt% to 30 wt%.

In the treatment with a salt, in particular, the treatment with a metal salt, it is preferable to perform the treatment in an acidic condition. When proanthocyanidins are treated in a condition of weak to strong alkalinity, the stability of proanthocyanidins is poor, so that proanthocyanidins may be decomposed. Thus, when using such a metal salt, it is preferable to adjust the pH of a solution prepared to have a concentration that is 2 to 10 times a predetermined final concentration to 4 to 6, preferably 4 to 5.5, and more preferably 4 to 5 in advance, and thereafter add this solution to the object to be treated.

After the salt is added, the mixture is allowed to stand at 1°C to 40°C for 30 minutes to 48 hours to deposit a sufficient amount of an insoluble substance such as a precipitate. Although the standing time may be longer than 48 hours, it is preferable to proceed to the next step before OPCs are automatically oxidized and the color of the mixture changes from reddish-brown to dark blackish brown.

Next, the resultant insoluble substance such as a precipitate is removed. As the method for removing the insoluble substance, any method commonly used by those skilled in the art, for example, filtration or centrifugation can be employed. In view of the treatment time, filtration is preferably used. Filtration can be performed preferably at 1°C to 40°C. The lower the temperature of filtration is, the more proanthocyanidins having a high degree of polymerization can be removed, so that filtration is performed preferably at 30°C or less, more preferably 25°C or less. The filtration may be performed before the salt is added, but it is necessary to perform filtration again after the addition of the salt in order to remove an insoluble substance such as a precipitate. Moreover, in order to minimize the loss of proanthocyanidins due to filtration, the residue remaining after the filtration can be washed with an aqueous solution having the same saturating concentration to obtain the washing liquid.

In the present invention, when the salt treatment is performed before the treatment with the adsorbents, the salt can be removed in the step of performing the treatment with the adsorbents, and thus a step of removing the salt is not necessarily required.

### (Proanthocyanidin-containing product)

The thus obtained proanthocyanidin-containing product contains proanthocyanidins at a high ratio. For the proanthocyanidins contained in the product, proanthocyanidins having a low degree of polymerization, preferably condensation products having a degree of polymerization of 2 to 30 (dimer to tridecamer), more preferably condensation products having a degree of polymerization of 2 to 10 (dimer to decamer), and even more preferably condensation products having a degree of polymerization of 2 to 4 (dimer to tetramer; OPCs) are preferable in view of bioactivities. The proanthocyanidin-containing product obtained by the method of the present invention especially contains OPCs at a high ratio, preferably in an amount of at least 35 wt%, more preferably at least 40 wt% in terms of dry weight. Furthermore, the ratio of the proanthocyanidins having a degree of polymerization of 5 or more is not more than 1 part by weight with respect to 1 part by weight of OPCs. Therefore, the proanthocyanidin-containing product is an excellent product having a high proanthocyanidin content with increased water solubility and bioactivities and decreased astringency and the like.

The ratio of OPCs in the total proanthocyanidins contained in the proanthocyanidin-containing product obtained by the present invention is preferably at least 45 wt%, and more preferably at least 50 wt%. Thus, the proanthocyanidin-containing product obtained by performing the treatment with at least two types of adsorbents has a higher OPC ratio in the total proanthocyanidins than the extract or juice of a plant or a product obtained by performing a treatment with a single adsorbent. Preferably, the OPC ratio is at least 3 times, and more preferably, at least 3.5 times higher than that of the extract or juice of a plant.

The proanthocyanidin-containing product obtained by the method of the present invention may further contain catechins preferably at 10 to 15 wt% in terms of dry weight. Catechins have poor water solubility and exhibit low bioactivities, but water solubility and bioactivities of catechins increase in the presence of OPCs. Therefore, a proanthocyanidin-containing product that contains OPCs and catechins is particularly useful.

The term "catechins" is a general term referring to polyhydroxyflavan-3-ols. Examples of the catechins include (+)-catechin, (-)-epicatechin, (+)-gallocatechin, (-)-epigallocatechin, epigallocatechin gallate, and epicatechin gallate. The catechins also include afzelechin and 3-galloyl derivatives of (+)-catechin or gallocatechin derived from natural products.

Examples of the effects of the catechins include a cancer inhibiting effect, an arteriosclerosis preventing effect, a fat metabolism disorder inhibiting effect, a blood pressure elevation inhibiting effect, a platelet aggregation inhibiting effect, an antiallergic effect, an antiviral effect, an antibacterial effect, a dental caries preventing effect, a halitosis preventing effect, an intestinal flora normalization effect, an active oxygen or free radical eliminating effect, an antioxidation effect, and an antidiabetic effect of inhibiting an elevation of blood glucose.

The proanthocyanidin-containing product having a very high OPC content obtained by the present invention can then be made into a concentrate, a dilution, and the like using a method commonly employed by those skilled in the art. In order to concentrate the product, various methods such as membrane concentration, heat concentration, vacuum (reduced pressure) concentration, and freeze concentration can be employed. Furthermore, the proanthocyanidin-containing product can be subjected to a sterilization treatment for storage as necessary. Sterilization can be performed by a method commonly used by those skilled in the art, such as stream sterilization, high pressure sterilization, or heat sterilization.

The proanthocyanidin-containing product may be concentrated, dried, and powdered after sterilization. Drying can be performed by a method commonly used by those skilled in the art. In particular, freeze drying, vacuum drying, and spray drying are preferably used.

The proanthocyanidin-containing product obtained in this manner can be used as a raw material of food products, pharmaceuticals, cosmetics, and quasi-drugs.

When utilized as the food products, the proanthocyanidin-containing product obtained by the present invention may be made into a health drink, gelled food and drink, and the like. Furthermore, the proanthocyanidin-containing product can be eaten or drunk as it is or with an additive mixed therewith. Examples of the additive include excipients, extenders, binders, thickeners, emulsifiers, flavors, nutritions, food additives, and seasonings. For example, the proanthocyanidin-containing product can be mixed with royal jelly, vitamins, proteins, calcium compounds, chitosan, lecithin, caffeine, and the like, which are the nutritions, and furthermore, sugar solutions and seasonings, which are the food additives, can be added thereto to control taste. The proanthocyanidin-containing product can be shaped as necessary into capsules such as hard capsules and soft capsules, tablets, or pills, or into the forms of powder, granule, candy, and the like. This product can be taken as it is, depending on the form or individual preference, or can be dissolved in water, hot water, milk, or the like or placed in a tea bag and infused in hot water or the like for drinking.

When the proanthocyanidin-containing product obtained by the present invention is utilized as the pharmaceuticals, it also can be shaped, as it is or using an additive and the like commonly used by those skilled in the art, into the forms similar to those of the food products described above.

When the proanthocyanidin-containing product obtained by the present invention is utilized as the cosmetics and the quasi-drugs, it can be shaped into the forms of ointment, cream, emulsion, lotion, skin lotion, pack, bath salt, and the like that are commonly used by those skilled in the art, because it has good solubility in aqueous solutions.

### Examples

Hereinafter, the present invention will be described by way of examples, but the present invention is not limited to these examples. The abbreviation "v/v" shown in the examples refers to "volume/volume", and "w/w" refers to "weight/weight".

### (Preparation of extract liquid)

First, 5.4 L of purified water was added to 1 kg of pine bark, and the pine bark was pulverized with a blender (Waring Blender). The pine bark was extracted under heating at 100°C for 24 hours while being refluxed. Immediately after that, the mixture was filtrated, and the resultant insoluble substances were washed with 1.6 L of purified water. The washing liquid was combined with the filtrated liquid to obtain 7 L of a pine bark extract liquid. When 10 mL of this extract liquid was freeze-dried, the dry weight was 70 mg. This extract liquid was allowed to cool to 25°C.

### (Example 1)

First, 1 L of the above-described extract liquid (dry weight of the powder derived from this extract liquid is 7 g) was applied on a column having a diameter of 5 cm and filled with 300 mL (corresponding to about 200 g) of Amberlite (registered trademark) XAD-4 (manufactured by ORGANO CORPORATION; an adsorbent that can remove proanthocyanidins having a degree of polymerization of 5 or more; the first adsorbent). Amberlite XAD-4 is an aromatic synthetic adsorbent that has a specific surface area of 700 m²/g and a pore radius of not more than 80 Å. Then, this column was washed with 600 mL of purified water. Furthermore, 800 mL of an 80 v/v% ethanol aqueous solution was applied on the column so that the adsorbate was eluted. The eluate was concentrated under reduced pressure to remove ethanol, and then water was added to the concentrate so that the volume of the resultant solution was 500 mL. Next, this solution was applied on a column having a diameter of 5 cm and filled with 200 mL (corresponding to about 140 g) of DIAION (registered trademark) HP-20 (manufactured by Mitsubishi Chemical Corporation; an adsorbent that can remove impurities; the second adsorbent). DIAION is an aromatic synthetic adsorbent that has a specific surface area of 600 m²/g and a pore radius of 100 to 120 Å. This column was washed with 600 mL of purified water. Thereafter, 200 mL of a 20 v/v% ethanol aqueous solution were applied on the column, and furthermore 50 mL of this aqueous solution were applied thereon. The resultant liquids were combined. The obtained liquid is taken as a proanthocyanidin-containing liquid A.

Next, in order to measure the amount of each component contained in the obtained proanthocyanidin-containing liquid A, the components in the proanthocyanidin-containing liquid A was separated into an OPC fraction, a fraction of proanthocyanidins having a degree of polymerization of 5 or more, a fraction containing catechins, and a fraction of other components than catechins in the following manner. First, the proanthocyanidin-containing liquid A was evaporated to dryness under reduced pressure, and the weight (the solid weight) of the resultant dry powder was measured. Then, 25 mL of Sephadex LH-20 (manufactured by Amersham Biotech) swollen with water were filled in a 15 x 300 mm column, and washed with 50 mL of ethanol. Next, 100 mg of the above-described dry powder were dissolved in 2 mL of ethanol, and this solution was applied on the column for adsorption. Thereafter, gradient elution was conducted using 100 to 80 v/v% ethanol-water mixed solvents, and the resultant eluate was collected in fractions of 10 mL each. When the fractions were collected, each of the fractions was subjected to silica gel thin layer chromatography (TLC) under the following conditions to detect whether or not OPCs were present, using specimens of dimeric to tetrameric OPCs (dimer: proanthocyanidin B-2 (Rf value: 0.6), trimer: proanthocyanidin C-1 (Rf value: 0.4), and tetramer: cinnamtannin A₂ (Rf value: 0.2)) as indicators.

TLC: silica gel plate (manufactured by Merck & Co., Inc.)

Eluent: benzene/methyl formate/formic acid (2/ 7 /1)

Detection reagent: a mixture of sulfuric acid and anisaldehyde

Amount of sample liquid: 10 µL each

The eluted fractions that were confirmed to contain OPCs by TLC were combined to obtain the OPC fraction.

Then, at the point when OPCs were not detected any more, 300 mL of a 50°/(v/v) water-acetone mixed solvent was applied on the column so that the remaining adsorbates that were adsorbed to the column were eluted.

The collected eluted fractions other than the OPC fraction were subjected to TLC and thus divided into a fraction (i.e., a combined fraction) containing catechins and a fraction (i.e., a combined fraction) containing proanthocyanidins having a degree of polymerization of 5 or more. The developing conditions of TLC and the detection method were as described above.

The fraction containing catechins was further divided into a fraction containing catechins and a fraction containing other components than catechins in the following manner. First, the fraction containing catechins was freeze-dried to obtain a powder. This powder was dissolved in 3 mL of water, and the resultant solution was applied on a 15 x 300 mm column filled with 20 mL of MCI Gel (manufactured by Mitsubishi Chemical Corporation) swollen with water for adsorption. This column was washed with water, and then gradient elution was conducted using 10 to 100%(v/v) ethanol-water mixed solvents, and the resultant eluate was collected in fractions of 7 mL each. After the elution, TLC was conducted to detect catechins in each of the fractions using a catechin as an indicator, and thus the fractions were divided into a catechin fraction (i.e., a combined catechin fraction) and a fraction (i.e., a combined fraction) of other components than catechins.

The thus obtained OPC fraction, fraction of proanthocyanidins having a degree of polymerization of 5 or more, catechin fraction, and fraction of other components than catechins were powdered by freeze drying, and the dry weight was measured. The total of the OPC fraction, the fraction of proanthocyanidins having a degree of polymerization of 5 or more, the catechin fraction, the fraction of other components than catechins, and the fraction of other components was 99.0 to 99.9 mg with respect to 100 mg of the dry powder of the proanthocyanidin-containing liquid A. This means that almost all the components had been recovered.

Table 1 shows the weight of solids in the proanthocyanidin-containing liquid A; the dry weights and contents of OPCs, proanthocyanidins having a degree of polymerization of 5 or more, the total proanthocyanidins (total of OPCs and proanthocyanidins having a degree of 5 or more), and catechins contained in the proanthocyanidin-containing liquid A; and the ratio of OPCs in the total proanthocyanidins. Table 1 further shows the conditions of the treatment with the adsorbents. Open circles indicate that the first or the second adsorbent was used, and crosses indicate that the first or the second adsorbent was not used.

### (Example 2)

The treatment was performed in the same manner as in Example 1, except that the treatment with the first adsorbent and the treatment with the second adsorbent were performed in reverse order, and thus a proanthocyanidin-containing liquid B was obtained. The obtained liquid was analyzed in the same manner as in Example 1. Table 1 shows the results.

### (Comparative Example 1)

The treatment was performed in the same manner as in Example 1, except that the treatment with the second adsorbent was not performed, and the obtained treated liquid was analyzed in the same manner as in Example 1. Table 1 shows the results.

### (Comparative Example 2)

The treatment was performed in the same manner as in Example 1, except that the treatment with the second adsorbent was not performed and a 20 v/v% ethanol aqueous solution was used instead of the 80 v/v% ethanol aqueous solution in the treatment with the first adsorbent, and the obtained treated liquid was analyzed in the same manner as in Example 1. Table 1 shows the results.

### (Comparative Example 3)

The treatment was performed in the same manner as in Example 1, except that the treatment with the first adsorbent was not performed, and the obtained treated liquid was analyzed in the same manner as in Example 1. Table 1 shows the results.

### (Comparative Example 4)

The treatment was performed in the same manner as in Example 1, except that the treatment with the first adsorbent was not performed and an 80 v/v% ethanol aqueous solution was used instead of the 20 v/v% ethanol aqueous solution in the treatment with the second adsorbent, and the obtained treated liquid was analyzed in the same manner as in Example 1. Table 1 shows the results.

### (Comparative Example 5)

The treatment was performed in the same manner as in Example 1, except that the treatment with the first adsorbent was not performed and a 5 v/v% ethanol aqueous solution was used instead of the 20 v/v% ethanol aqueous solution in the treatment with the second adsorbent, and the obtained treated liquid was analyzed in the same manner as in Example 1. Table 1 shows the results.

### (Comparative Example 6)

The treatment was performed in the same manner as in Example 1, except that the treatment with the first adsorbent was performed again instead of performing the treatment with the second adsorbent, and the obtained treated liquid was analyzed in the same manner as in Example 1. Table 1 shows the results.

### (Comparative Example 7)

The treatment was performed in the same manner as in Example 1, except that the treatment with the second adsorbent was performed again instead of performing the treatment with the first adsorbent, and the obtained treated liquid was analyzed in the same manner as in Example 1. Table 1 shows the results.

### (Comparative Example 8)

An extract liquid from pine bark was analyzed, instead of the proanthocyanidin-containing liquid A, in the same manner as in Example 1. Table 1 shows the results.

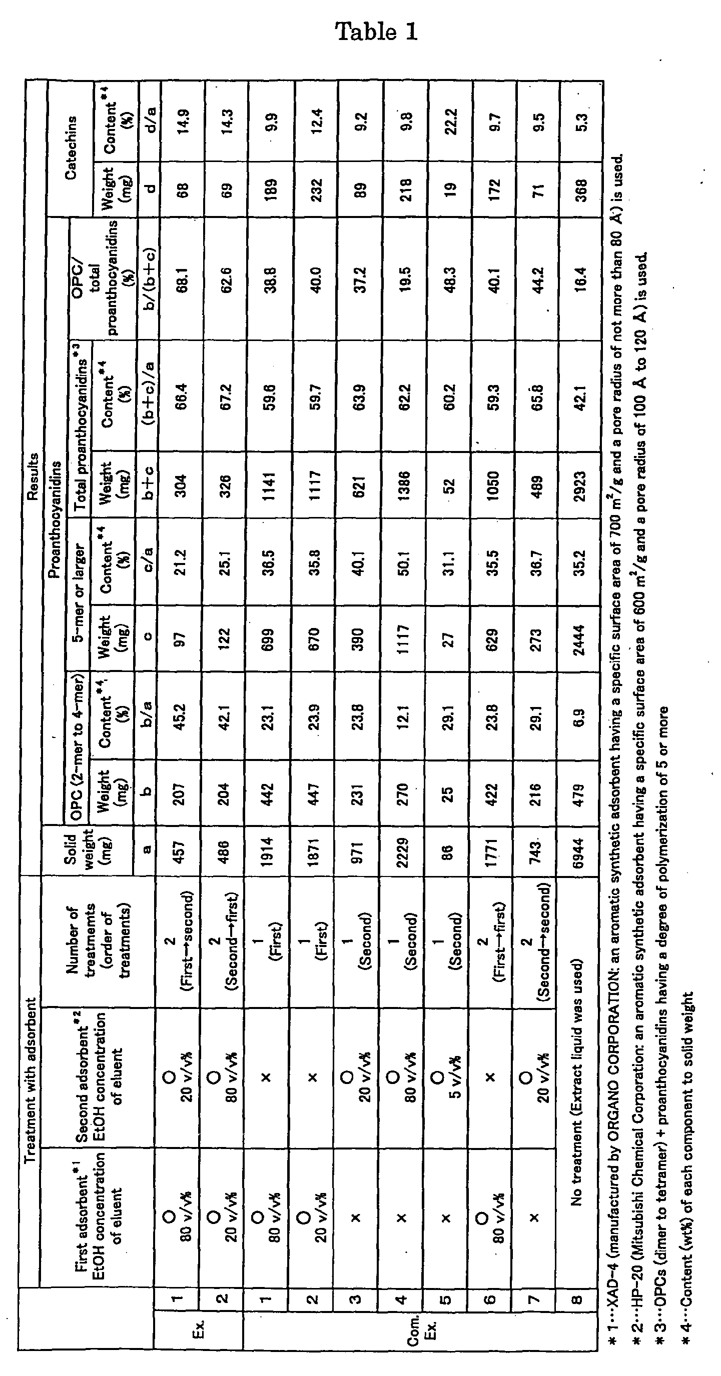

From the results in Table 1, it is found that in Examples 1 and 2 in which two different types of adsorbents were used, a proanthocyanidin-containing product containing OPCs at a high ratio can be obtained. In particular, it is found that in Examples 1 and 2, since the treatment was performed using the adsorbent for eluting proanthocyanidins having a degree of polymerization of 5 or more and removing these proanthocyanidins (the first adsorbent) and the adsorbent for removing impurities and obtaining OPCs in fractions (the second adsorbent) in combination, the resultant dry powder contains OPCs at a ratio of 40 wt% or more.

The adsorbents used in Comparative Examples 1 and 4 especially differ from one another in the pore radius. In Comparative Example 1 in which the adsorbent having a pore radius of not more than 90 Å was used, the OPC content is higher than in Comparative Example 4 in which the adsorbent having a pore radius of not less than 100 Å was used. On the other hand, in Comparative Example 4, the content of the total proanthocyanidins in the dry powder is higher than that in Comparative Example 1. Thus, when an adsorbent having a pore radius of not more than 90 Å is used for conducting adsorption of proanthocyanidins, proanthocyanidins having a degree of polymerization of 5 or more are easily removed. Therefore, the amount of OPCs contained in the resultant treated product is relatively high. When an adsorbent having a pore radius of not less than 100 Å is used, the content of the total proanthocyanidins in the resultant treated product tends to be relatively high. By comparison between Comparative Examples 1 and 2, it is found that when an adsorbent having a pore radius of not more than 90 Å is used, the OPC content does not vary even with different elution solvents. By comparison between Comparative Examples 3 and 4, it is found that when an adsorbent having a pore radius of not less than 100 Å is used, the OPC content varies with different elution solvents. From this, it is considered that an adsorbent having a pore radius of not less than 100 Å is suitably used when OPCs are to be selectively eluted depending on the concentration of the solvent. Comparative Example 5 aims to obtain a proanthocyanidin-containing product having a high OPC content by separating OPCs from proanthocyanidins having a degree of polymerization of 5 or more with the second adsorbent alone. In Comparative Example 5, the elution solvent (5 v/v% ethanol aqueous solution) that satisfies a condition that OPCs alone are eluted is used, but it is found that OPCs are not eluted sufficiently and the amount of OPCs themselves that can be recovered is low.

### (Example 3)

First, 2 L of purified water was added to 100 g of pine bark, the pine bark was pulverized with a blender (Waring Blender), and then the mixture was heated at 95°C for one hour. Then, immediately after that, the mixture was filtrated, and the resultant insoluble substances after the filtration were washed with 1 L of purified water. The washing liquid was combined with the filtrated liquid to obtain 3 L of an extract liquid.

Then, 70 g (corresponding to about 100 mL) of SEPABEADS SP850 were added to 3 L of this extract liquid, and the mixture was stirred for 3 hours. Thereafter, the mixture was filtrated to obtain a solid substance to which proanthocyanidins were adsorbed. This solid substance was washed with 250 mL of purified water, and 150 mL of an 80 (v/v)% ethanol aqueous solution were added thereto. The resultant mixture was stirred for one hour and then filtrated to obtain a filtrated liquid. This filtrated liquid was concentrated under reduced pressure to remove ethanol, and purified water was added thereto so that the volume of the resultant solution was 1 L. Then, 70 g of DIAION (registered trademark) HP-20 were further added to this solution, and the mixture was stirred for 3 hours and thereafter filtrated to obtain a solid substance to which proanthocyanidins were adsorbed. This solid substance was washed with 250 mL of purified water, and 150 mL of a 15 (v/v)% ethanol aqueous solution were added thereto. The resultant mixture was stirred for one hour and then filtrated to obtain a filtrated liquid. This filtrated liquid was freeze-dried, and thus 351 mg of a proanthocyanidin-containing powder were obtained. Then, 100 mg of this proanthocyanidin-containing dry powder was taken to measure the contents of OPCs and catechins in the same manner as in Example 1. The dry powder contained 42.3 wt% of OPCs, 10 wt% of proanthocyanidins having a degree of polymerization of 5 or more, and 14.9 wt% of catechins in terms of dry weight.

### (Example 4)

First, 1 L of purified water was added to 100 g of pine bark, the pine bark was pulverized with a blender (Waring Blender), and then the mixture was heated at 100°C for 12 hours. Then, immediately after that, the mixture was filtrated, and the resultant insoluble substances after the filtration were washed with 200 mL of purified water. The washing liquid was combined with the filtrated liquid to obtain 1.2 L of an extract liquid. This extract liquid was applied on a column filled with 80 g (corresponding to about 100 mL) of Amberlite (registered trademark) XAD-4. This column was washed with 300 mL of purified water, and then 600 mL of a 20 (v/v)% ethanol aqueous solution were applied on the column to obtain an eluate. The eluate was evaporated to dryness under reduced pressure, and the resultant powder was dissolved in 500 mL of anhydrous ethanol. A column filled with 300 mL of Sephadex LH-20 swollen with water was washed with 300 mL of ethanol, and then the above-described eluate was applied on the column. This column was washed with 600 mL of anhydrous ethanol, and then 1.5 L of an 80 v/v% ethanol aqueous solution was applied thereon to obtain a proanthocyanidin-containing liquid. This proanthocyanidin-containing liquid was evaporated to dryness under reduced pressure, and thus 421 mg of a proanthocyanidin-containing product was obtained. When the contents of OPCs and catechins were measured in the same manner as in Example 1, the product contained 46.2 wt% of OPCs, 15.2 wt% of proanthocyanidins having a degree of polymerization of 5 or more, and 11.1 wt% of catechins in terms of dry weight.

### Industrial Applicability

According to the method of the present invention, proanthocyanidin-containing products containing highly bioactive OPCs at a high ratio can be obtained efficiently and easily by treating an extract or juice of a plant with at least two types of adsorbents that differ from one another in at least one of the material, the specific surface area, the pore radius, and the ability of adsorbing and releasing a substance based on the molecular weight of the substance. These proanthocyanidin-containing products are effective for improving vascular proliferation, hypertension, oversensitiveness to the cold and the like, and are very useful as a raw material for producing food products, pharmaceuticals, cosmetics, and quasi-drugs.

## Claims

1. A method for producing a proanthocyanidin-containing product, comprising the step of treating an extract or juice of a plant with at least two types of adsorbents,
wherein the adsorbents differ from one another in at least one of material, pore radius, specific surface area, and an ability of adsorbing and releasing a substance based on the molecular weight of the substance.

2. The method of claim 1, wherein at least one of the adsorbents is a synthetic adsorbent.

3. The method of claim 1 or 2, which is performed using two types of adsorbents, wherein a first adsorbent is a synthetic adsorbent, and a second adsorbent is selected from the group consisting of a synthetic adsorbent, a cation exchange resin, an anion exchange resin, a crosslinked dextran derivative, a polyvinyl resin, an agarose derivative, and a cellulose derivative.

4. The method of claim 1 or 2, wherein at least one of the adsorbents can remove a proanthocyanidin having a degree of polymerization of 5 or more or impurities from the extract or juice of a plant.

5. The method of any one of claims 1 to 3, wherein at least one of the adsorbents is porous and has a pore radius of not more than 90 Å or not less than 100 Å.

6. The method of any one of claims 1 to 4, wherein at least one of the adsorbents has an ability of adsorbing and releasing a substance having the molecular weight in the range of 100 to 20000.
